**Europäisches Patentamt**

**European Patent Office**

**Office européen des brevets**

(19)

(11) Veröffentlichungsnummer : **0 235 217 B2**

(12) **NEUE EUROPÄISCHE PATENTSCHRIFT**

(45) Veröffentlichungstag der neuen Patentschrift :
**27.10.93 Patentblatt 93/43**

(51) Int. Cl.$^5$ : **A61K 7/13**

(21) Anmeldenummer : **86905258.9**

(22) Anmeldetag : **07.08.86**

(86) Internationale Anmeldenummer :
**PCT/EP86/00466**

(87) Internationale Veröffentlichungsnummer :
**WO 87/01033 26.02.87 Gazette 87/05**

(54) **VERFAHREN UND MITTEL ZUM OXIDATIVEN FÄRBEN VON HAAREN.**

Die Akte enthält technische Angaben, die nach dem Eingang der Anmeldung eingereicht wurden und die nicht in dieser Patentschrift enthalten sind.

(30) Priorität : **24.08.85 DE 3530270**

(43) Veröffentlichungstag der Anmeldung :
**09.09.87 Patentblatt 87/37**

(45) Bekanntmachung des Hinweises auf die Patenterteilung :
**28.11.90 Patentblatt 90/48**

(45) Bekanntmachung des Hinweises auf die Entscheidung über den Einspruch :
**27.10.93 Patentblatt 93/43**

(84) Benannte Vertragsstaaten :
**FR GB NL**

(56) Entgegenhaltungen :
BE-A-83 253 3
DE-A- 2 222 001
FR-A- 1 439 307
JP-A- 4 418 599
JP-A-53 072 836
JP-A-53 130 443
JP-A-53 133 641
US-A- 2 934 396
CHEMICAL ABSTRACTS, Band 89, Nr. 20, 13. November 1978, Columbus, Ohio, USA; Seite 347, Zusammenfassung 168955b; & JP-A-7872836
CHEMICAL ABSTRACTS, Band 90, Nr. 14, 2. April 1979, Columbus, Ohio, USA; Seite 373, Zusammenfassung 109799b; & JP-A-78133641
Demande de brevet allemande S 119187, als Zusammenfassunf J.Saphir (1937)
The Chemistry of synthetic dyes-vol.V. (1971) chapter VII, Seiten 475-495
Oxidation in organic chemistry (1973), Teil B, Kapitel II, Seiten 103-108

(56) Entgegenhaltungen :
**Advanced Inorganic Chemistry, 4. Auflage, 1980, Seiten 738-749**
**Metal-Catalyzed Oxidations of Organic Compounds (1081), Kapitel V, Seite 145**
**Problemes Capillaires (1966)**
**Lehrbuch der Anorganischen Chemie (1964), 180-181, 536-537 (Holleman-Wiberg)**
**H.Römpp Chemielexikon, Seite 604-606**
**Preislisten der Kabinettartikel 1975,76,83,84**
**E.Sagarin, Cosmetics (1957), Seiten 503-505**
**H.Janistyn, Band III (1973), Seiten 388-397**
**"aucola"-Gebrauchsanweisung und Versuchsergebnisse; pH-Wert Bestimmung datiert 06.06.91**
**J.C. Johnson "Hair Dyes", Noyes Data Corporation (1973), Seiten 94-111 und Seiten 250-255**
**Ullmanns Encyklopädie der technischen Chemie, 3. Auflage, 10. Band, Seiten 727,734,741 und 742**
**J.S. Jellinek: Kosmetologie, Dr. Alfred Hüthig Verlag, Heidelberg, 3. Auflage, 1976, Seite 659**
**K.H. Schrader: Grundlagen und Rezepturen der Kosmetika, Dr. Alfred Hüthig Verlag, Heidelberg, 1. Auflage 1979, Seite 533**
**K.H. Schrader: Grundlagen und Rezepturen der Kosmetika, Dr. Alfred Hüthig Verlag, Heidelberg, 2. Auflage 1989, Seiten 796-797**
**Prospekte und Verkaufszahlen der Goldwell AG**

(73) Patentinhaber : **GOLDWELL AKTIENGESELLSCHAFT**
**Zerninstrasse 10-18**
**D-64297 Darmstadt (DE)**

(72) Erfinder : **TENNIGKEIT, Jürgen**
**Felsbergstrasse 51**
**D-6104 Seeheim 2 (DE)**
Erfinder : **LORENZ, Herbert**
**Ober-Ramstädterstrasse 22**
**D-6101 Gross-Bieberau (DE)**

EP 0 235 217 B2

**Beschreibung**

Die Erfindung betrifft ein Verfahren zum oxidativen Färben von menschlichen oder tierischen Haaren, bei dem unmittelbar vor der Anwendung aus wenigstens einem Oxidationsfarbstoff und einem Oxidationsmittel ein Haarfärbemittel aufbereitet, auf das zu färbende Haar aufgetragen und eine vorgegebene Zeitdauer zur Einwirkung gebracht wird, worauf das Haarfärbemittel ausgewaschen wird, sowie ein Haarfärbemittel zur Durchführung des Verfahrens.

Für die dauerhafte Färbung von menschlichem Haar werden alkalische Oxidationsfarbstoffe in Pastenform verwendet, die unmittelbar vor der Anwendung mit einem sauren Oxidationsmittel, z.B. Wasserstoffperoxid, zum gebrauchsfertigen, auf das zu färbende Haar aufzutragende Haarfärbepräparat vermischt werden. Die Färbung entsteht hierbei durch Reaktion bestimmter Entwicklersubstanzen mit bestimmten Kupplersubstanzen in Gegenwart eines geeigneten Oxidationsmittels. Das gebrauchsfertige Präparat liegt dabei deutlich im alkalischen Bereich, nämlich i.a. bei pH-Werten von 8,5 bis 10,5, wodurch die Oberflächen der zu färbenden Haare in einer für das Eindringen der Oxidationsstoffe vorteilhaften Weise aufgeschlossen werden. Andererseits beanspruchen alkalische Präparate die Haare auch, so daß es-insbesondere bei wiederholten Einwirkungen, z.B. bei mehrfachem Nachfärben-zu Schädigungen des Haares kommen kann. Solche Nachfärbungen sind aber-insbesondere bei hellergefärbtem Haar-erforderlich, um beispielsweise den ungefärbten Haarnachwuchs farblich an die bereits gefärbten Haarlängen anzugleichen und die früher gefärbten Haarlängen farblich aufzufrischen, wenn sie im Laufe der Zeit durch äußere Einwirkungen z.B. Sonnenbestrahlung, häufiges Waschen u.dgl. gegenüber der ursprünglichen Farbe aufgehellt sind.

Versuche, die insbesondere in den Haarspitzen auftretenden schädigenden Wirkungen der alkalischen Haarfärbemittel durch saure Einstellung des aus dem Oxidationsfarbstoff und dem Oxidationsmittel fertig aufbereiteten Präparate zu vermeiden, führten zu einer ungenügenden Anfärbung der Haare und wurden deshalb nicht weiter verfolgt.

Aus der DE-A-2 222 001 ist eine Formulierung bekannt, über deren pH-Wert nichts ausgesagt ist. Es handelt sich dabei um ein Haarfärbemittel, das aus mindestens einem Oxidationsfarbstoff, Wasser, einem Lösungsmittel, einer Säure oder Base zur Einstellung des (nicht genannten) pH-Werts, gegebenenfalls einem Verdickungs- und einem Treibmittel und zusätzlich einer Metallkomplexverbindung als Katalysator besteht. Dabei sind vorzugsweise Metallkomplexverbindungen von Cu, Fe, Mn, Co, Ni, Cr, Ti, Hf, Sn, Zn, V, Zr und Mo genannt. Das Mittel soll sich durch besonders einfache Handhabung auszeichnen und wird auf dem Haar belassen. Nachteilig ist, daß der Oxidationsvorgang ohne gezielte Zugabe von Oxidationsmittel völlig unkontrolliert verläuft. Auf dem Haar verbleibende Chemikalien strapazieren das Haar unnötig.

Aus der FR-A-1 439 307 ist ein Haarfärbemittel und -verfahren bekannt, bei dem organisch komplexierte Metallchelate verwendet werden. Das Mittel besteht im wesentlichen aus einem Oxidationsfarbstoff, einem Oxidationsmittel und einem organischen Chelatkomplex der Gruppe Cr, Mn, Fe, Co, Ni, Cu. Aus den Beispielen geht jedoch hervor, daß die Lösung mit konzentriertem Ammoniak alkalisch gemacht wird. Es tritt also wiederum das Problem auf, daß die Haare, besonders bei wiederholter Anwendung, geschädigt werden.

Der Erfindung liegt demgegenüber die Aufgabe zugrunde, ein Verfahren zum oxidativen Färben von Haaren sowie das hierzu erforderliche Haarfärbemittel anzugeben, welche-auch bei mehrfacher Anwendung-nicht zu den geschilderten Haar-Schädigungen führen.

Ausgehend von einem Verfahren der eingangs erwähnten Art wird diese Aufgabe erfindungsgemäß dadurch gelöst, daß ein aus Oxidationsfarbstoff und Oxidatiomsmittel aufbereitetes, auf einen schwach sauren pH-Wert im Bereich von 5,9 bis 6,9 eingestelltes Haarfärbemittel verwendet wird, und daß dem Oxidationsfarbstoff vor, zugleich mit oder unmittelbar nach der Vermischung mit dem Oxidationsmittel Mangandioxid in Pulverform in solcher Menge zugegeben wird, daß es im gebrauchsfertigen Haarfärbemittel mit einem Anteil zwischen 0,02 und 5 Gew.%, vorzugsweise zwischen 0,12 und 0,18 Gew.% enthalten ist. Es hat sich gezeigt, daß bei Verwendung eines derartigen schwach sauer eingestellten Haarfärbemittel im Beisein von Mangandioxid hervorragend haltbare Färbungen von menschlichem Haar in brillanten Farbtönen möglich sind, sofern nicht dunkleres Haar auf einen helleren Farbton umgefärbt werden soll. Die gegenüber den früheren Versuchen mit sauren Haarfärbemitteln erfolgreiche Färbewirkung wird auf die katalytische Wirkung des im Haarfärbemittel enthaltenen Mangandioxids zurückgeführt. Des weiteren wurde festgestellt, daß die auf die alkalische Einstellung der bisher verwendeten Haarfärbemittel zurückzuführenden Haarschädigungen vollständig vermieden werden.

Aus den vorstehenden Hinweisen geht hervor, daß für die aufhellende oder hellerziehende Färbung von dunklerem Haar auf die bekannten alkalischen Haarfärbemittel zurückgegriffen werden muß. Da Schädigungen des Haares durch solche alkalischen Haarfärbemittel aber in der Begel erst bei mehrfachem Nachfärben auftreten, können solche Schäden vermieden werden, wenn zum Nachfärben von im Naturzustand dunklerem hellgefärbtem Haar so verfahren wird, daß zunächst auf die dunkleren, nachgewachsenen kopthautnahen Haarlängen ein bekanntes, alkalisch eingestelltes Haarfärbemittel, auf die bereits früher gefärbten anschließenden Haarlängen jedoch das schwach saure Haarfärbemittel aufgebracht wird, und daß nach hinreichender Einwirkungszeit sowohl das alkalische als auch das schwach saure Haarfärbemittel aus dem behandelten Haar ausgewaschen werden.

Besonders problematisch in bezug auf Haarschädigungen ist eine kombinierte Dauerwell- und Haarfärbebehandlung.

Wesentlich verringert wird die Gefahr von Haarschäden bei einem Verfahren zum Wellen und oxidativen Färben von Haaren, bei dem auf das gewaschene und auf Wicklern aufgewickelte Haar zunächst ein Dauerwellpräparat für eine vorgegebene Zeitdauer zur Einwirkung gebracht und dann ausgewaschen wird, worauf das noch aufgewickelte Haar mit einem flüssigen oxidierenden Fixiermittel vorfixiert wird und schließlich das von den Wicklern befreite Haar in einem weiteren Behandlungsschritt mit einem oxidierenden Fixiermittel nachfixiert wird, dadurch, daß für die Nachfixierung das schwach saure Haarfärbemittel verwendet, für die zur hinreichenden Einfärbung der Haare erforderliche Zeitdauer auf dem Haar zur Einwirkung gebracht und anschließend ausgewaschen wird. Das Haarfärbemittel ersetzt also das beim Dauerwellen üblicherweise beim Nachfixieren verwendete Fixiermittel, d.h. wird sozusagen in Doppelfunktion eingesetzt, wobei die sonst bei Verwendung von alkalisch eingestellten Haarfärbemitteln zu befürchtenden Schädigungen vermieden sind.

Das bei der Durchführung des erfindungsgemäßen Verfahrens verwendete unmittelbar vor der Anwendung durch Vermischen eines Oxidationsfarbstoffs mit einem Oxidationsmittel hergestellte Präparat ist also dadurch gekennzeichnet, daß es auf einen pH-Wert zwischen 5,9 bis 6,9 eingestellt ist und zusätzlich pulverförmiges Mangandioxid in feiner Verteilung enthält.

Die Menge des Mangandioxid ist dabei vorzugsweise so bemessen, daß es im Haarfärbemittel in Anteilen zwischen 0,02 und 5 Gew.%, vorzugsweise zwischen 0,12 und 0,18 Gew.%, enthalten ist.

Beispiel 1a

Oxidationsfärbung

20 ml Färbepaste der unten angegebenen Rezeptur für die Farbe "Haselnußblond" mit einem Zusatz von 0,12% Mangan(IV)oxid in feinverteilter Form wurde mit 40 ml einer 2% $H_2O_2$ enthaltenden Entwicklerlösung vermischt, woraus ein Haarfärbemittel mit einem pH-Wert von 6,7 resultierte. Das Mittel wurde auf ein mittelstarkes Haar mit Grundton mittelaschblond aufgetragen und 20 Minuten zur Einwirkung gebracht.

Als Ergebnis wurde eine Umfärbung des Haares zu einem intensiven Haselnußblond-Farbton erhalten.

Versuche ergaben, daß die Einwirkungszeit des Haarfärbemittels bei entsprechend verringerter resultierender Farbintensität bis auf 5 Minuten verkürzt werden kann.

Rezeptur der Färbepaste "Haselnußblond":

| | | |
|---|---|---|
| Cetylstearylalkohol | 10,0 | g |
| Kokosfettsäuremonoäthanolamid | 2,0 | g |
| Stearinsäuremonoäthanolamid | 2,0 | g |
| Stearinsäurediäthanolamid | 1,0 | g |
| | | |
| p-Toluylendiaminsulfat | 0,25 | g |
| m-Aminophenol | 0,01 | g |
| Resorcin | 0,01 | g |
| p-Aminophenol | 0,08 | g |
| p-Amino-o-kresol | 0,06 | g |
| Pikraminsäure | 0,05 | g |
| Monoäthanolamin | 0,2 | g |
| Ammoniumchlorid | 0,2 | g |
| Natriumlaurylsulfat | 0,3 | g |
| Mangan(IV)oxid | 0,12 | g |
| Natriumsulfit | 0,25 | g |
| Äthylendiamintetraessigsäure | 0,2 | g |
| Parfum | 0,2 | g |
| Enth. Wasser auf | 100,00 | g |

Beispiel 1b

Oxidationsfärbung

20 ml Färbepaste der unten angegebenen Rezeptur für die Farbe "Kupferblond" mit einem Zusatz von 0,12% Mangan(IV)oxid in feinverteilter Form wurde mit 40 ml einer 2% $H_2O_2$ enthaltenden Entwicklerlösung vermischt, woraus ein Haarfärbemittel mit einem pH-Wert von 6,7 resultierte.

Das Mittel wurde auf ein mittelstarkes Haar mit dem Grundton mittelaschblond aufgetragen und 20 Minuten zur Einwirkung gebracht.

Als Ergebnis wurde eine Umfärbung des Haars in einen dezenten Kupferton erhalten.

Versuche ergaben, daß die Einwirkungszeit des Haarfärbemittels bei entsprechend verringerter resultierender Farbintensität bis auf 5 Minuten verkürzt werden kann.

Rezeptur der Färbepaste "Kupferblond":

| | | |
|---|---|---|
| Cetylstearylalkohol | 10,0 | g |
| Kokosfettsäuremonoäthanolamid | 2,0 | g |
| Stearinsäuremomoäthanolamid | 2,0 | g |
| Stearinsäurediäthanolamid | 1,0 | g |
| p-Toluylendiaminsulfat | 0,20 | g |
| p-Aminophenol | 0,70 | g |
| p-Amino-o-kresol | 0,70 | g |
| Monoäthanolamin | 0,2 | g |
| Ammoniumchlorid | 0,2 | g |
| Natriumlaurylsulfat | 0,3 | g |
| Mangan(IV)oxid | 0,12 | g |
| Natriumsulfit | 0,12 | g |
| Äthylendiamintetraessigsäure | 0,2 | g |
| Parfum | 0,2 | g |
| Enth. Wasser auf | 100,00 | g |

Beispiel 2

Oxidationsfärbung zum schonenden, sauren Ausgleich der Haarspitzen nach normaler, alkalischer Ansatzfärbung

20 ml Färbepaste der beim Beispiel 1a angegebenen Rezeptur für die Farbe "Haselnußblond" mit einem Zusatz von 0,12% Mangan(IV)oxid in feinverteilter Form wurde mit 40 ml einer 2% $H_2O_2$ enthaltenden Entwicklerlösung vermischt, woraus ein Haarfärbemittel mit einem pH-Wert von 6,7 resultierte.

Das Mittel wurde auf ein mittelstarkes Haar, welches haselnußblond gefärbt war und dessen Längen und Spitzen ausgeblichen sind und einen Ton von hellaschblond haben, aufgetragen und 10 Minuten zur Einwirkung gebracht.

Als Ergebnis wurde ein dem Ansatz angeglichener Haselnußblond-Farbton der ausgeblichenen Längen und Spitzen erhalten. Die Einwirkungsdauer des Präparats kann bei geringeren auszugleichenden Farbintensitäten bis auf 5 Minuten verkürzt werden.

Beispiel 3a

Oxidationsfärbung in Verbindung mit der Dauerwellung

Auf mittelstarkem Haar, das einen Grundton von dunkelgoldblond hatte und dessen Haarlängen und -spitzen ausgeblichen waren, wurde eine Dauerwellung durchgeführt.

Die Dauerwelle wurde bis einschließlich der ersten Fixierung auf dem Wickler nach Gebrauchsanweisung verarbeitet.

Zur Nachfixierung und zum gleichzeitigen Färben wurde nach dem Entfernen der Wickler 20 ml Färbepaste der unten angegebenen Rezeptur für die Farbe "Zyklamen" mit einem Zusatz von 0,12% Mangan(IV)oxid in feinverteilter Form mit 40 ml einer Dauerwell-Fixierung mit einem $H_2O_2$-Gehalt von 2% vermischt.

Das hieraus resultierende Haarfärbemittel mit einem pH-Wert von 6,7 wurde nun mittels einer Auftrageflasche bzw. eines Schwämmchens auf das Haar aufgetragen und 10 Minuten zur Einwirkung gebracht. Als Ergebnis wurde zusätzlich zur Dauerwellung eines Umfärbung des Haars in einen rotvioletten (Zyklamen)-Farbton erhalten. Je nach gewünschter Farbintensität kann die Einwirkungsdauer zwischen 5 und 20 Minuten variiert werden.

Rezeptur der Färbepaste "Zyklamen":

| | | |
|---|---|---|
| Cetylstearylalkohol | 10,0 | g |
| Kokosfettsäuremonoäthanolamid | 2,0 | g |
| Stearinsäuremonoäthanolamid | 2,0 | g |
| Stearinsäurediäthanolamid | 1,0 | g |
| p-Toluylendiaminsulfat | 0,5 | g |
| p-Amino-o-kresol | 0,4 | g |
| p-Aminophenol | 0,1 | g |
| Monoäthanolamin | 0,2 | g |
| Ammoniumchlorid | 0,2 | g |
| Natriumlaurylsulfat | 0,3 | g |
| Mangan(IV)oxid | 0,12 | g |
| Natriumsulfit | 0,25 | g |
| Äthylendiamintetraessigsäure | 0,2 | g |
| Parfum | 0,2 | g |
| Enth. Wasser auf | 100,00 | g |

Beispiel 3b

Oxidationsfärbung in Verbindung mit der Dauerwellung

Auf mittelstarkem Haar, das einen Grundton von dunkelgoldblond hatte und dessen Haarlängen und -spitzen ausgeblichen waren, wurde eine Dauerwellung durchgeführt.

Die Dauerwelle wurde bis einschließlich der ersten Fixierung auf dem Wickler nach Gebrauchsanweisung verarbeitet.

Zur Nachfixierung und zum gleichzeitigen Färben wurde nach dem Entfernen der Wickler 20 ml Färbepaste der unten angegebenen Rezeptur für die Farbe "Mahagoni" mit einem Zusatz von 0,12% Mangan(IV)oxid in feinverteilter Form mit 40 ml einer Dauerwell-Fixierung mit einem $H_2O_2$-Gehalt von 2% vermischt.

Das hieraus resultierende Haarfärbemittel mit einem pH-Wert von 6,7 wurde nun mittels einer Auftrageflasche bzw. eines Schwämmchens auf das Haar aufgetragen und 10 Minuten zur Einwirkung gebracht. Als Ergebnis wurde zusätzlich zur Dauerwellung eine Umfärbung des Haars in einen kräftigen Rotton erhalten. Je nach gewünschter Farbintensität kann die Einwirkungsdauer zwischen 5 und 20 Minuten variiert werden.

Rezeptur der Färbepaste "Mahagoni":

| | | |
|---|---:|---|
| Cetylstearylalkohol | 10,0 | g |
| Kokosfettsäuremonoäthanolamid | 2,0 | g |
| Stearinsäuremonoäthanolamid | 2,0 | g |
| Stearinsäurediäthanolamid | 1,0 | g |
| p-Toluylendiaminsulfat | 0,4 | g |
| p-Aminophenol | 0,4 | g |
| p-Amino-o-kresol | 0,4 | g |
| o-Nitro-p-Phenylendiamin | 0,4 | g |
| Monoäthanolamin | 0,2 | g |
| Ammoniumchlorid | 0,2 | g |
| Natriumlaurylsulfat | 0,3 | g |
| Mangan(IV)oxid | 0,12 | g |
| Natriumsulfit | 0,25 | g |
| Äthylendiamintetraessigsäure | 0,2 | g |
| Parfum | 0,2 | g |
| Enth. Wasser auf | 100,00 | g |

## Patentansprüche

1. Verfahren zum oxidativen Färben von menschlichen oder tierischen Haaren, bei dem unmittelbar vor der Anwendung aus wenigstens einem aus Entwickler- und Kupplersubstanzen entstehenden Oxidationsfarbstoff und einem Oxidationsmittel, vorzugsweise Wasserstoffperoxid, ein Haarfärbemittel aufbereitet, auf das zu färbende Haar aufgetragen und eine vorgegebene Zeitdauer zur Einwirkung gebracht wird, worauf das Haarfärbemittel ausgewaschen wird, dadurch gekennzeichnet, daß ein aus Oxidationsfarbstoff und Oxidationsmittel aufbereitetes, auf einen schwach sauren pH-Wert im Bereich von 5,9 bis 6,9 eingestelltes Haarfärbemittel verwendet wird, und daß dem Oxidationsfarbstoff vor, zugleich mit oder unmittelbar nach der Vermischung mit dem Oxidationsmittel Mangandioxid in Pulverform in solcher Menge zugegeben wird, daß es im gebrauchsfertigen Haarfärbemittel mit einem Anteil zwischen 0,02 und 5 Gew.%, vorzugsweise zwischen 0,12 und 0,18 Gew.% enthalten ist.

2. Verfahren nach Anspruch 1 zum Nachfärben von im Naturzustand dunklerem heller gefärbtem Haar, dadurch gekennzeichnet, daß zunächst auf die dunkleren nachgewachsenen kopfhautnahen Haarlängen ein handelsübliches alkalisches Haarfärbemittel und dann das schwach saure Haarfärbemittel auf die bereits früher gefärbten anschließenden Haarlängen aufgebracht wird, und daß nach hinreichender Einwirkungszeit sowohl das alkalische als auch das schwach saure Haarfärbemittel aus dem behandelten Haar ausgewaschen werden.

3. Verfahren zum Wellen und oxidativen Färben von Haaren nach Anspruch 1, bei dem auf das gewaschene und auf Wicklern aufgewickelte Haar zunächst ein Dauerwellpräparat für eine vorgegebene Zeitdauer zur Einwirkung gebracht und dann ausgewaschen wird, worauf das noch aufgewickelte Haar mit einem flüssigen oxidierenden Fixiermittel vorfixiert wird, und schließlich das von den Wicklern befreite Haar in einem weiteren Behandlungsschritt mit einem oxidierenden Fixiermittel nachfixiert wird, dadurch gekennzeichnet, daß für die Nachfixierung das schwach saure Haarfärbemittel verwendet, für die zur hinreichenden Einfärbung der Haare erforderliche Zeitdauer auf dem Haar zur Einwirkung gebracht und anschließend ausgewaschen wird.

4. Mittel für die oxidative Färbung vor menschlichen oder tierischen Haaren, welches unmittelbar vor der Anwendung durch Vermischen eines aus Entwickler- und Kupplersubstanzen entstehenden Oxidationsfarbstoffs mit einem Oxidationsmittel hergestellt ist, dadurch gekennzeichnet, daß das Mittel auf einen pH-Wert zwischen 5,9 und 6,9 eingestellt ist und zusätzlich pulverförmiges Mangandioxid in Anteilen zwischen 0,02 und 5 Gew.%, vorzugsweise zwischen 0,12 und 0,18 Gew.% in feiner Verteilung enthält.

## Claims

1. Process for the oxidative dyeing of human or animal hair in which, immediately before the application, a hair dye is prepared from at least one oxidation dye being produced from developing and coupling substances and an oxidant, preferably hydrogen peroxide, applied to the hair to be colored, and allowed to act for a given period of time, whereupon the hair dye is washed out, characterized in that a hair dye prepared from oxidation dye and oxidant, and adjusted to a weakly acid pH value in the range from 5.9 to 6.9, is used, and that manganese dioxide in powder form is added to the oxidation dye before, simultaneously with or immediately after mixing with the oxidant, in such an amount that it is contained in the ready-to-use hair dye in a percentage between 0.02 to 5 wt%, preferably between 0.12 and 0.18 wt%.

2. Process according to claim 1 for the redyeing of lighter colored hair that is darker in the natural state, characterized in that first a commercial alkaline hair dye is applied to the darker, regrown hair lengths, close to the scalp, and then the weakly acid hair dye is applied to the previously dyed adjoining hair lengths, and that, after sufficient time of action, both the alkaline and the weakly acid hair dye are washed out of the treated hair.

3. Process for the waving and oxidative dyeing of hair according to claim 1, in which at first a permanent-wave preparation is made to act for a given time on the hair that has been washed and wound on curlers, and it is then washed out, whereupon the still wound hair is pre-fixed with a liquid oxidizing fixer, and lastly the hair, freed from the curlers, is post-fixed in an additional treatment step with an oxidizing fixer, characterized in that the weakly acid hair dye is used for the post-fix, is made to act on the hair for the period necessary for the sufficient coloring of the hair, and is then washed out.

4. Substance for the oxidative dyeing of human or animal hair, which is prepared immediately before use by mixing an oxidation dye, being produced from developing and coupling substances, with an oxidant, characterized in that the substance is adjusted to a pH value between 5.9 and 6.9 and additionally contains powdered manganese dioxide in finely divided form, in percentages between 0.02 and 5 wt%, preferably between 0.12 and 0.18 wt%.

## Revendications

1. Procédé pour la teinture par oxydation des cheveux ou des poils dans lequel, immédiatement avant l'application, on prépare une teinture pour cheveux ou poils à partir d'au moins un colorant par oxydation se formant d'agents de développement et couplage et d'un agent oxydant, de préférence le peroxyde d'hydrogène, on l'applique sur les cheveux ou poils à teindre et on laisse agir pendant une durée déterminée puis on élimine la teinture par lavage, caractérisé en ce que l'on utilise une teinture pour cheveux ou poils préparée à partir d'un colorant par oxydation et d'un agent oxydant, réglée à un pH légèrement acide dans l'intervalle de 5,9 à 6,9 et en ce que l'on ajoute au colorant par oxydation avant, pendant ou immédiatement après le mélange avec l'agent oxydant, du bioxyde de manganèse à l'état de poudre, en quantité telle que la teinture prête à l'emploi en contienne de 0,02 à 5 % en poids, de préférence de 0,12 à 0,18 % en poids.

2. Procédé selon la revendication 1 pour les retouches de teinture de cheveux ou poils de couleur sombre à l'état naturel et teints en nuances plus claires, caractérisé en ce que l'on applique d'abord sur les parties des cheveux proches du cuir chevelu, fraîchement repoussées et de couleur sombre, une teinture pour cheveux alcaline du commerce et on applique ensuite sur les longueurs des cheveux qui ont été teintes antérieurement la teinture pour cheveux légèrement acide et, après une durée d'action suffisante, on élimine à la fois la teinture alcaline légèrement acide par lavage des cheveux ou poils traités.

3. Procédé pour l'ondulation et la teinture par oxydation des cheveux selon la revendication 1, dans lequel, sur les cheveux lavés et enroulés sur des bigoudis, on fait d'abord agir un produit pour ondulation permanente pendant une durée déterminée puis on l'élimine par lavage, on soumet les cheveux toujours enroulés sur bigoudis à une fixation préalable à l'aide d'un fixateur liquide oxydant, et finalement, après avoir retiré les bigoudis et dans une autre opération, on soumet la chevelure à une fixation complémentaire par un fixateur oxydant, ce procédé se caractérisant en ce que, pour la fixation complémentaire, on utilise la teinture pour cheveux légèrement acide, on la laisse agir pendant la durée suffisante pour parvenir à la

7

coloration voulue puis on l'élimine par lavage.

4. Produit pour la teinture par oxydation des cheveux ou des poils, préparé immédiatement avant l'application par mélange d'un colorant par oxydation se formant d'agents de développement et couplage et d'un agent oxydant, caractérisé en ce que le produit est réglé à un pH de 5,9 à 6,9 et contient en outre du bioxyde de manganèse à l'état de poudre en proportions de 0,02 à 5 %, de préférence de 0,12 à 0,18 %, à l'état de fine division.